# FASCICULE DE BREVET EUROPEEN

(11) **EP 1 553 942 B1**
(45) Date de publication et mention de la délivrance du brevet: **24.05.2006**
(21) Numéro de dépôt: 03778425.3
(22) Date de dépôt: 21.10.2003
(51) Int. Cl.: A61K 31/444, A61K 31/616, A61K 31/60, A61K 31/196, A61K 31/407, A61K 31/405, A61K 31/192, A61K 31/19, A61K 31/5415, A61K 31/34, A61K 31/365, A61K 31/415, A61K 31/635, A61P 29/00, A61P 19/02, A61P 25/00

(54) **COMPOSITION PHARMACEUTIQUE ASSOCIANT LE TENATOPRAZOLE ET UN ANTI-INFLAMMATOIRE**
PHARMAZEUTISCHE ZUSAMMENSETZUNG ENTHALTEND TENATOPRAZOL UND EINEN ENTZÜNDUNGSHEMMER
PHARMACEUTICAL COMPOSITION COMBINING TENATOPRAZOLE AND AN ANTI-INFLAMMATORY AGENT

(30) Priorité: 21.10.2002 FR 0213115
(43) Date de publication de la demande: 20.07.2005
(73) Titulaire: Sidem Pharma SA, 2441 Luxembourg (LU); Mitsubishi Pharma Corporation, Chuo-ku, Tokyo 103-8405 (JP)
(72) Inventeur: SCHUTZE, François, F-78860 SAINT-NOM-LA-BRETECHE (FR); CHARBIT, Suzy, F-94000 CRETEIL (FR); FICHEUX, Hervé, F-94130 NOGENT-SUR-MARNE (FR); HOMERIN, Michel, F-91080 COURCOURONNES (FR); TACCOEN, Alain, F-78150 LE CHESNAY (FR); INABA, Yoshio, Tokyo 103-8405 (JP)
(74) Mandataire: L'Helgoualch, Jean
(86) Numéro de dépôt international: PCT/FR2003/003120
(87) Numéro de publication internationale: WO 2004/037254

(56) Documents cités:
- WO-A-01/56573
- WO-A-01/66088
- WO-A-02/22108
- US-A1- 2002 119 977

## Description

La présente invention concerne une nouvelle composition utilisable en thérapeutique, et plus particulièrement une nouvelle composition pharmaceutique combinant un anti-inflammatoire et le ténatoprazole, pour le traitement symptomatique de la douleur et des maladies inflammatoires en évitant les effets secondaires des anti-inflammatoires usuels.

Les anti-inflammatoires constituent une classe de médicaments largement utilisés depuis de nombreuses années. L'un des premiers anti-inflammatoires utilisés en thérapeutique est l'aspirine, dont les propriétés antipyrétiques, antalgiques et anti-agrégantes plaquettaires sont également bien connues et justifient son administration dans diverses indications. Ainsi, on estime que plusieurs millions de comprimés d'aspirine sont consommés chaque année dans le monde.

Les anti-inflammatoires non stéroïdiens (AINS) sont les médicaments le plus largement utilisés dans le traitement de la douleur et de l'inflammation aiguë. Ils se répartissent essentiellement entre les AINS classiques et les inhibiteurs des iso-enzymes-2 de la cyclo-oxygénase (COX-2).

Par exemple, l'aspirine, le diclofénac, l'étodolac, l'indométacine, le naproxène, l'ibuprofène et le piroxicam, sont des AINS classiques couramment prescrits pour le traitement symptomatique des rhumatismes inflammatoires et des arthroses.

Cependant, les AINS classiques présentent certains effets indésirables, et en particulier une tendance à induire des ulcérations gastriques ou intestinales (Goodman and Gilman, The Pharmacological Basis of Therapeutics; 9ème édition, McGraw Hill). Ces effets indésirables sont liés à l'inhibition de l'enzyme cyclo-oxygénase-1 (COX-1), isoforme constitutive. Ils sont d'autant plus gênants que le médicament doit être administré sur une période de temps plus longue, en particulier dans le traitement des affections chroniques.

La découverte de l'existence d'une autre isoforme de l'enzyme cyclo-oxygénase, la cyclo-oxygénase-2 (COX-2), isoforme induite dans l'établissement de l'inflammation, a permis d'envisager la mise au point de médicaments potentiellement plus spécifiques et plus sûrs. Ces AINS disponibles aujourd'hui ont pour effet d'inhiber sélectivement l'action de la COX-2, et donc d'agir sur l'inflammation avec une plus faible incidence d'effets secondaires indésirables sur le tractus gastro-intestinal supérieur. Ainsi, des inhibiteurs de COX-2, tels que le célécoxib et le rofécoxib, ont été développés et constituent une nouvelle classe de médicaments pour le traitement symptomatique des maladies inflammatoires.

Toutefois, si les inhibiteurs de COX-2 permettent de réduire sensiblement les troubles majeurs, tels que les ulcères gastriques ou hémorragiques, liés à l'administration des AINS classiques, ils n'apportent pas d'amélioration significative en ce qui concerne les troubles mineurs tels que les gastralgies et les dyspepsies, et ils n'évitent pas tous les troubles majeurs. Ainsi, une étude récente a montré que les pourcentages de troubles mineurs chez des patients recevant du célécoxib étaient de 4,8% (gastralgies), 4,8% (symptômes dyspeptiques) et 2,4% (nausées), tandis que dans le cas d'un traitement par un AINS classique ces pourcentages sont respectivement de 6,2%, 5,9% et 3,4%. Des résultats semblables sont obtenus en comparant les traitements avec un autre inhibiteur de COX-2, le rofécoxib, et des AINS classiques.

Le ténatoprazole, c'est-à-dire la 5-méthoxy-2-[[(4-méthoxy-3,5-diméthyl-2-pyridyl)méthyl]sulfinyl]imidazo[4,5-b]pyridine, est décrit dans le brevet EP 254.588. Il fait partie des médicaments considérés comme des inhibiteurs de la pompe à protons, utiles pour le traitement des ulcères gastriques et duodénaux.

Le premier dérivé connu de cette série est l'oméprazole, décrit dans le brevet EP 005.129, qui possède des propriétés inhibitrices de la sécrétion acide gastrique, et est largement utilisé comme anti-ulcéreux en thérapeutique humaine. Parmi les autres inhibiteurs de la pompe à protons, outre l'oméprazole, on peut citer aussi le rabéprazole, le pantoprazole, le lansoprazole, qui présentent tous une analogie structurelle, et se rattachent au groupe des pyridinyl-méthyl-sulfinyl-benzimidazoles. Le ténatoprazole présente une structure analogue, mais du type imidazo-pyridine. Ces composés sont des sulfoxydes présentant une asymétrie au niveau de l'atome de soufre et sont donc généralement sous forme de mélange racémique de deux énantiomères.

L'oméprazole a aussi été envisagé dans le traitement des troubles du reflux gastro-oesophagien, mais son action dans une telle indication n'est pas totalement satisfaisante. Ainsi, des études ont montré que sa durée d'action, comme dans le cas des autres inhibiteurs de la pompe à protons, est insuffisante pour traiter efficacement le reflux nocturne.

Le ténatoprazole est décrit en détail dans le brevet EP 254.588, ainsi que ses propriétés inhibitrices de l'ATPase (H⁺ + K⁺) et de la sécrétion d'acide gastrique.

On a déjà proposé de prescrire des inhibiteurs de la pompe à protons, tels que l'oméprazole, à des patients traités par des anti-inflammatoires afin de limiter les effets secondaires et en particulier les complications liées aux lésions et ulcères gastriques, mais ces effets secondaires des anti-inflammatoires peuvent être très sévères et sont imprévisibles, notamment chez les sujets à risques tels que les personnes âgées, et l'administration conjointe d'un inhibiteur usuel de la pompe à protons ne répond pas de manière satisfaisante à la nécessité d'un traitement préventif.

La demande de brevet WO 01.66088 concerne une forme pharmaceutique autoémulsionnante pour administration orale d'un AINS libérant le groupement NO, formant *in situ* une émulsion au contact des fluides gastriques. Il évoque aussi la possibilité d'associer un tel anti-inflammatoire et un inhibiteur usuel de la pompe à protons, tel que l'oméprazole. La demande de brevet WO 01.56573 décrit des anti-inflammatoires de la série des inhibiteurs de COX2 qui sont supposés augmenter la motilité gastro-intestinale, et il envisage lui aussi de les associer à des inhibiteurs de la pompe à protons. Toutefois, aucun de ces documents ne décrit un seul exemple d'une telle association et n'envisage plus particulièrement d'associer un anti-inflammatoire avec spécifiquement le ténatoprazole.

L'association d'un analogue de la prostaglandine E1 tel que le misoprostol à un anti-inflammatoire tel que le diclofénac et a aussi été proposée pour traiter les effets secondaires d'ulcération gastrique de l'anti-inflammatoire, mais la demi-vie d'élimination du misoprostol est trop courte pour procurer un effet prolongé.

Il subsiste donc un besoin d'un médicament à activité anti-inflammatoire qui puisse être utilisé dans des traitements prolongés sans entraîner d'effet secondaire néfaste, notamment chez les patients âgés ou présentant des risques d'ulcération gastro-duodénale, et en procurant en outre un effet préventif vis-à-vis de tels effets secondaires.

La présente invention a précisément pour objet de mettre à la disposition des praticiens un médicament destiné au traitement des manifestations douloureuses et inflammatoires, notamment au traitement symptomatique des maladies inflammatoires telles que, par exemple, les rhumatismes inflammatoires, l'arthrite et l'arthrose, et exerçant un effet préventif vis-à-vis des effets secondaires de lésions et d'ulcérations gastroduodénales.

Les études réalisées par la demanderesse ont montré que l'association du ténatoprazole et d'un anti-inflammatoire procure des effets inattendus par rapport aux autres inhibiteurs de la pompe à protons et aux anti-inflammatoires, notamment les AINS, utilisés isolément ou en association. Plus particulièrement, il a été montré que l'association du ténatoprazole et d'un ou plusieurs anti-inflammatoires procure un contrôle de l'acidité gastrique combiné à l'action anti-inflammatoire procurant une efficacité améliorée et une meilleure sécurité d'emploi, et permet de traiter efficacement des patients souffrant de douleurs et de maladies inflammatoires, en particulier d'inflammations rhumatismales telles que l'arthrite, la polyarthrite rhumatoïdes et l'arthrose en évitant les troubles digestifs induits par les anti-inflammatoires.

La présente invention a donc pour objet une composition pharmaceutique associant un inhibiteur de la pompe à protons spécifique, le ténatoprazole, et un ou plusieurs anti-inflammatoires .

La présente invention a aussi pour objet une composition pharmaceutique pour administration par voie orale ou parentérale, comprenant du ténatoprazole et un ou plusieurs anti-inflammatoires, sous une forme adaptée au traitement symptomatique des manifestations douloureuses et inflammatoires.

L'invention a encore pour objet l'utilisation combinée du ténatoprazole et d'au moins un anti-inflammatoire pour le traitement symptomatique des manifestations douloureuses et inflammatoires, ainsi que l'utilisation combinée du ténatoprazole et d'au moins un anti-inflammatoire pour la fabrication d'un médicament destiné au traitement symptomatique des manifestations douloureuses et inflammatoires.

Suivant l'invention, le ténatoprazole peut être utilisé sous forme libre ou sous forme de sel, et par exemple de sel de potassium, de magnésium, de sodium ou de calcium.

L'anti-inflammatoire utilisé dans les compositions suivant la présente invention peut être choisi parmi les anti-inflammatoires non stéroïdiens (AINS) classiques et les inhibiteurs de la cyclo-oxygénase-2. Ainsi, on peut associer le ténatoprazole et l'aspirine, ou un AINS classique choisi parmi le diclofénac, l'étodolac, l'indométacine, le naproxène, l'ibuprofène et le piroxicam. L'inhibiteur de cyclo-oxygénase-2 utilisé dans les compositions de l'invention peut être par exemple le célécoxib ou le rofécoxib.

Les compositions conformes à la présente invention peuvent être avantageusement utilisées, comme indiqué ci-dessus, dans tous les traitements des manifestations douloureuses et inflammatoires, en particulier chez les sujets âgés, les sujets présentant des antécédents ulcéreux, les patients sous traitement par de l'aspirine ou des anticoagulants, etc. Elles conviennent notamment au traitement des rhumatismes inflammatoires, en particulier l'arthrite et l'arthrose, des douleurs gingivales, etc, où elles évitent les complications digestives majeures et mineures liées aux anti-inflammatoires connus.

Les études effectuées par la demanderesse ont montré que ces symptômes peuvent être traités efficacement avec une composition conforme à la présente invention, associant le ténatoprazole et un anti-inflammatoire, et que l'avantage constitué par le moindre risque d'effets secondaires, notamment de lésions et d'ulcères gastroduodénaux, résulte d'une forme d'activité spécifique du ténatoprazole complétant celle de l'anti-inflammatoire.

En effet, le ténatoprazole se distingue des autres inhibiteurs de la pompe à protons par une demi-vie d'élimination étonnamment plus longue, et aussi par une exposition tissulaire importante, comme l'ont démontré les expérimentations effectuées par la demanderesse.

Ainsi, l'étude de phase I chez des individus de type causasien (n=8 par groupe) a permis de montrer l'influence de différentes doses de ténatoprazole sur les paramètres pharmacocinétiques, dans le cas d'une administration par voie orale en une dose unique, et pendant une période de 7 jours.

Les doses testées sont de 10, 20, 40 et 80 mg de ténatoprazole.

Les résultats obtenus sont regroupés au Tableau 1 ci-après.

**Tableau 1**

| | Dose unique | | | | Dose répétée (7 jours) | | | |
|---|---|---|---|---|---|---|---|---|
| | 10 mg | 20 mg | 40 mg | 80 mg | 10 mg | 20 mg | 40 mg | 80 mg |
| Cmax (µg/ml) | 0,9 | 2,4 | 5,3 | 8,3 | 1,6 | 3 | 5,5 | 11,8 |
| Tmax (h) | 4 | 4 | 3 | 3 | 3 | 2 | 3 | 2 |
| T1/2 (h) | 5 | 6 | 6 | 7 | 5 | 8 | 9 | 9,2 |
| AUC 0-t | 8 | 24 | 43 | 97 | 13 | 36 | 75 | 218 |

Dans ce tableau, les abréviations utilisées ont les significations suivantes :
- Cmax: concentration maximale
- Tmax: temps pour obtenir la concentration maximale
- T1/2: temps de demi-vie d'élimination
- AUC₀₋ₜ: aire sous la courbe, entre le temps 0 et la dernière concentration mesurable.

Les résultats exposés au Tableau 1 ci-dessus montrent que les moyennes de temps de demi-vie d'élimination sont comprises entre 5 et 6 heures après administration d'une dose unique, et entre 5 et 9,5 heures après 7 jours d'administration, selon la dose. Le ténatoprazole présente aussi de fortes valeurs d'AUC (aire sous la courbe) mettant en évidence un faible taux de métabolisme et/ou une forte biodisponibilité par voie orale. De plus, quelles que soient les conditions d'administration, unique ou répétée, les valeurs de Cmax, AUC₀₋ₜ et AUC_{0-inf} augmentent de manière linéaire. La valeur de AUC_{0-inf} est calculée par extrapolation.

Une comparaison des valeurs d'AUC entre deux inhibiteurs de la pompe à protons, le lansoprazole et l'oméprazole, a déjà été faite par Tolman et al. (J. Clin. Gastroenterol., 24(2), 65-70, 1997) mais elle ne permet pas de juger de la supériorité d'un produit par rapport à un autre. En effet, différents critères entrent en jeu, à savoir le temps de régénération de la pompe, et le temps passé au-dessus de la concentration minimale nécessaire pour inhiber les pompes à protons. En ce qui concerne le temps de régénération des pompes, on observe que les pompes ont généralement une durée de demi-vie de l'ordre de 30 à 48 heures, et elles sont donc renouvelées totalement toutes les 72 à 96 heures.

L'étude pharmacocinétique réalisée par la demanderesse a montré que, grâce aux propriétés pharmacocinétiques inattendues exposées ci-dessus, le ténatoprazole permet de s'opposer au phénomène de régénération des pompes à protons en maintenant une concentration inhibitrice sur une période de temps suffisamment longue pour répondre aux deux critères précités.

Ainsi, l'exposition prolongée liée à la longue demi-vie d'élimination du ténatoprazole, mise en évidence par la valeur d'AUC, lui confère une plus longue présence au niveau des sites d'action et procure donc un effet pharmacodynamique prolongé dans le temps. Les expérimentations montrent ainsi que le ténatoprazole possède un rapport demi-vie plasmatique /temps de régénération des pompes notablement plus élevé que celui des autres inhibiteurs de la pompe à protons, ce qui permet de l'utiliser dans des pathologies où les médicaments actuels sont peu efficaces, en particulier dans le traitement des symptômes nocturnes du reflux gastro-oesophagien, ainsi que des ulcères gastriques et duodénaux.

Aussi, lorsqu'il est associé à un anti-inflammatoire, tel que le diclofénac, le célécoxib, l'indométacine, le naproxène, l'ibuprofène et le rofécoxib, de préférence par administration le soir au coucher, le ténatoprazole, par comparaison avec les autres inhibiteurs de la pompe à protons, procure un avantage significatif en ce qui concerne la suppression de l'acidité gastrique, et par conséquent permet une action efficace sur le pic nocturne d'acidité gastrique ainsi que sur les symptômes nocturnes chez les patients souffrant de reflux gastro-oesophagien, auquel il procure un soulagement important, même chez les patients réfractaires aux traitements classiques par des inhibiteurs de la pompe à protons usuels tels que l'oméprazole.

La composition de la présente invention peut être administrée sous les formes usuelles adaptées au mode d'administration choisi, par exemple par voie orale ou parentérale, de préférence par voie orale ou intraveineuse. On peut utiliser par exemple des formulations de comprimés ou de gélules contenant le ténatoprazole et l'anti-inflammatoire comme principes actifs, ou encore des émulsions ou solutions pour administration parentérale contenant un sel de ténatoprazole associé à un ou plusieurs anti-inflammatoires, avec un support pharmaceutiquement acceptable usuel.

Les doses unitaires peuvent contenir entre 10 et 60 mg de ténatoprazole et entre 10 et 500 mg d'anti-inflammatoire, en particulier le diclofénac, le naproxène, l'ibuprofène, le célécoxib ou le rofécoxib. Le rapport en poids du ténatoprazole à l'anti-inflammatoire doit être compris entre 1 : 2 et 1:40.

A titre d'exemple, une formulation appropriée de gélule contenant du ténatoprazole associé à un anti-inflammatoire non stéroïdien classique, est indiquée ci-dessous :

| | | |
|---|---|---|
| Ténatoprazole | | 20 mg |
| Diclofénac | | 100 mg |
| excipients | q.s.p. | 300 mg |

Un exemple de formulation associant du ténatoprazole et un inhibiteur de cyclo-oxygénase est indiqué ci-dessous :

| | | |
|---|---|---|
| Ténatoprazole | | 20 mg |
| Célécoxib | | 200 mg |
| excipients | q.s.p. | 300 mg |

La posologie est déterminée par le praticien en fonction de l'état du patient et de la gravité de l'affection. Elle est généralement comprise entre 10 et 120 mg, de préférence entre 20 et 40 mg, de ténatoprazole par jour, pour 20 à 1.600 mg d'anti-inflammatoire.

Par exemple, un traitement d'une poussée inflammatoire douloureuse d'arthrose du genou chez un sujet âgé peut consister en l'administration de 1 à 2 comprimés contenant chacun 20 mg de ténatoprazole et 100 mg de diclofénac, chaque soir pendant une période de temps qui peut être comprise entre 4 et 10 semaines, dans le cas d'un traitement d'attaque ou d'entretien.

Dans le cas d'affections sévères, il peut être efficace d'administrer le médicament dans un premier temps par voie intraveineuse, puis par voie orale. L'invention présente en outre l'avantage de permettre un traitement séquentiel efficace par simple administration, par semaine, d'un seul comprimé dosé à 20 ou 40 mg de ténatoprazole associé à 100 à 200 mg d'anti-inflammatoire par exemple le diclofénac, le célécoxib ou le rofécoxib.

L'étude de cas cliniques décrite ci-après a mis en évidence l'efficacité de la composition de l'invention.

**Tableau 2 Prévention des troubles digestifs**

| Age/sexe | Association | Rapport | Durée de | Trouble | Trouble | Tolérance |
|---|---|---|---|---|---|---|
| | AINS / ténato. | en poids | traite- | digestif | digestif | |
| | | | ment | grave | mineur | |
| 45/F | Naproxène / T | 500/20 | 8 sem. | 0 | 0 | +++ |
| 39/F | Diclofénac / T | 100/20 | 12 sem. | 0 | 0 | +++ |
| 41/M | Ibuprofène / T | 1600/20 | 8 sem. | 0 | 0 | +++ |
| 34/M | Diclofénac / T | 100/20 | 8 sem. | 0 | 0 | +++ |
| 52/M | Célécoxib / T | 200/20 | 8 sem. | 0 | 0 | +++ |
| 39/M | Célécoxib / T | 200/20 | 10 sem. | 0 | 0 | +++ |

| | | | | | | |
|---|---|---|---|---|---|---|
| T = ténatoprazole | | | | | | |

Le rapport en poids de l'AINS au ténatoprazole est exprimé en mg. Ainsi "Naproxène / T" "500/20" signifie une gélule associant 500 mg de naproxène et 20 mg de ténatoprazole. Le traitement comprenait l'administration d'une gélule par jour pendant la duré indiquée. Dans le cas de l'association de l'ibuprofène et du ténatoprazole, chaque gélule contenait 400 mg d'ibuprofène et 5 mg de ténatoprazole, et l'administration se faisait à raison de 4 gélules par jour.

Les résultats exposés au Tableau 2 ci-dessus montrent que l'administration d'une compositions suivant l'invention, associant le ténatoprazole à un anti-inflammatoire non stéroïdien, n'a entraîné aucun trouble digestif, grave ou mineur, et que le traitement a été très bien toléré.

## Revendications

1. Composition pharmaceutique pour le traitement des manifestations douloureuses et inflammatoires **caractérisée en ce qu'**elle comprend en combinaison le ténatoprazole et un ou plusieurs anti-inflammatoires choisis parmi
- les anti-inflammatoires non stéroïdiens choisis parmi l'aspirine, le diclofénac, l'étodolac, l'indométacine, le naproxène, l'ibuprofène et le piroxicam, et
- les inhibiteurs de la cyclo-oxygénase-2 choisis parmi le rofécoxib et le célécoxib.

2. Composition selon la revendication 1, **caractérisée en ce que** le rapport en poids du ténatoprazole à l'anti-inflammatoire est compris entre 1:2 et 1:40.

3. Composition selon l'une quelconque des revendications précédentes, **caractérisée en ce qu'**elle comprend des doses unitaires contenant entre 10 et 60 mg de ténatoprazole et entre 10 et 500 mg d'anti-inflammatoire.

4. Composition selon la revendication 1, **caractérisée en ce que** le ténatoprazole est sous forme de sel de potassium, de magnésium, de sodium ou de calcium.

5. Composition selon l'une quelconque des revendications précédentes, **caractérisée en ce qu'**elle est présentée sous une forme pour administration orale ou parentérale.

6. Utilisation combinée du ténatoprazole et d'au moins un anti-inflammatoire tel que défini dans la revendication 1 pour la fabrication d'un médicament destiné au traitement des manifestations douloureuses et inflammatoires.

## Claims

1. Pharmaceutical composition for the treatment of pain and inflammatory diseases, **characterized in that** it comprises a combination of tenatoprazole and one or more anti-inflammatory agents selected from
- nonsteroidal anti-inflammatory agents selected from aspirin, diclofenac, etodolac, indometacin, naproxen, ibuprofen and piroxicam, and
- cyclo-oxygenase-2 inhibitors selected from rofecoxib and celecoxib.

2. Composition according to claim 1, **characterized in that** the weight ratio tenatoprazole : anti-inflammatory agent is between 1:2 et 1:40.

3. Composition according to any one of the preceding claims, **characterized in that** the composition comprises unitary doses each containing from 10 to 60 mg of tenatoprazole and from 10 to 500 mg of anti-inflammatory agent.

4. Composition according to claim 1, **characterized in that** tenatoprazole is in the form of a potassium, magnesium, sodium or calcium salt.

5. Composition according to any one of the preceding claims, **characterized in that** it is administrable via the oral or the parenteral route.

6. The combined use of tenatoprazole and one or more anti-inflammatory agents according to claim 1 for the manufacture of a medicament for the treatment of pain and inflammatory diseases.

## Patentansprüche

1. Pharmazeutische Zusammensetzung zur Behandlung von Schmerzen und Entzündungen, **dadurch gekennzeichnet, dass** sie eine Kombination aus Tenatoprazol und einem oder mehreren entzündungshemmenden Mitteln, ausgewählt aus
- nicht-steroiden entzündungshemmenden Mitteln, ausgewählt aus Aspirin, Diclofenac, Etodolac, Indometacin, Naproxen, Ibuprofen und Piroxicam, und
- Inhibitoren von Cyclooxygenase-2, ausgewählt aus Rofecoxib und Celecoxib, umfasst.

2. Zusammensetzung nach Anspruch 1, **dadurch gekennzeichnet, dass** das Gewichtsverhältnis zwischen Tenatoprazol und dem entzündungshemmenden Mittel zwischen 1:2 und 1:40 liegt.

3. Zusammensetzung nach einem der vorangehenden Ansprüche, **dadurch gekennzeichnet, dass** sie Einheitsdosen umfasst, die zwischen 10 und 60 mg Tenatoprazol und zwischen 10 und 500 mg entzündungshemmendes Mittel enthalten.

4. Zusammensetzung nach Anspruch 1, **dadurch gekennzeichnet, dass** Tenatoprazol in Form von Kalium-, Magnesium-, Natrium- oder Calciumsalz vorliegt.

5. Zusammensetzung nach einem der vorangehenden Ansprüche, **dadurch gekennzeichnet, dass** sie in einer Form zur oralen oder parenteralen Verabreichung vorliegt.

6. Kombinierte Verwendung von Tenatoprazol und zumindest einem entzündungshemmenden Mittel wie in Anspruch 1 definiert zur Herstellung eines Medikaments zur Behandlung von Schmerzen und Entzündungen.
